# EUROPEAN PATENT APPLICATION

(11) **EP 3 792 367 A1**
(43) Date of publication of application: **17.03.2021**
(21) Application number: 19196672.0
(22) Date of filing: 11.09.2019
(51) Int. Cl.: C12Q 1/6844, C12N 15/86

(54) **METHOD FOR THE PRODUCTION OF RAAV AND METHOD FOR THE IN VITRO GENERATION OF GENETICALLY ENGINEERED, LINEAR, SINGLE-STRANDED NUCLEIC ACID FRAGMENTS CONTAINING ITR SEQUENCES FLANKING A GENE OF INTEREST**

(71) Applicant: Universität Bielefeld, 33615 Bielefeld (DE)
(72) Inventor: MÜLLER, Kristian, 14469 Potsdam (DE); RADUKIC, Marco, 33334 Gütersloh (DE)
(74) Representative: Gramm, Lins & Partner Patent- und Rechtsanwälte PartGmbB

(57) **Abstract**

In a first aspect, the present invention relates to a method for the *in vitro* generation of genetically engineered, linear, single-stranded nucleic acid fragments containing two viral inverted terminal repeat (ITR) sequences flanking a gene of interest (GOI). The method is based on a rolling circle amplification. In a further aspect, the present invention provides an isolated, linear, single-stranded nucleic acid comprising viral nucleic acid fragments obtainable by a method according to present invention. Further, a method for the production of recombinant virus particles, in particular, recombinant AAV particles (rAAV) based on the linear, single-stranded nucleic acid fragments is described herein. Moreover, a plasmid comprising specific nucleic acid sequence elements is provided.

## Description

In a first aspect, the present invention relates to a method for the *in vitro* generation of genetically engineered, linear, single-stranded nucleic acid fragments containing two viral inverted terminal repeat (ITR) sequences flanking a gene of interest (GOI). The method is based on a rolling circle amplification. In a further aspect, the present invention provides an isolated, linear, single-stranded nucleic acid comprising viral nucleic acid fragments obtainable by a method according to present invention. Further, a method for the production of recombinant virus particles, in particular, recombinant AAV particles (rAAV) based on the linear, single-stranded nucleic acid fragments is described herein. Moreover, a plasmid comprising specific nucleic acid sequence elements is provided.

### Prior art

Adeno-associated virus (AAV), in particular, serotype 2, has gained tremendous popularity as vector for gene therapy, but also as platform for vaccine development and as a tool in pre-clinical research. That is, virus derived vectors represent one of the most popular gene delivery systems for mammalian cells. The interest in AAV vector particles is based on several beneficial features including apathogenicity, high stability, ability to transduce both dividing and non-dividing cells, long term gene expression in post-mitotic or slowly proliferating cells and low immunogenicity. In addition, recombinant AAV vectors lack an intrinsic integrase activity and are therefore defined as non-integrating vector systems. This is a main advantage compared to retro-/lentiviral vectors as it significantly reduces the risk of insertional mutagenesis. AAV vectors are typically produced from mammalian or insect cell cultures following sophisticated protocols.

Research and development of recombinant Adeno-associated virus (rAAV) vectors for clinical applications is coming more and more in focus raising interest in suitable techniques to produce rAAV preparations at high titer and improved purity. The development is boosted by the recent approval of three AAV-based gene therapeutics for orphan diseases by regulatory bodies in the EU and the United States, namely Glybera, Luxturna and Zolgensma. AAV-based therapeutics for indications with higher prevalence, including hemophilia, are subject of ongoing clinical trials. In addition, basic research discloses more and more fields of application, for example, site directed gene therapy with rAAVs or tumor directed gene therapy.

One major drawback of rAAVs therapeutics is the complex production method using biological agents in living cells required to mimic a lytic viral infection. Typically, immortalized, kidney-derived human cells are used to produce rAAV. Cultivation thereof is expensive and prone to failure. One possible risk arises from potential co-infection of the producer cells with other human or primate viruses. Depleting viral contaminants in the product is difficult, in particular, when the final therapeutic product is a virus like particle (VLP) or viral vector. Another problem is the presence of bacterial nucleic acid sequences packaged into AAV capsids (Chadeuf, G., et. Al., 2005, Molecular Therapy: The Journal of the American Society of Gene Therapy, 12(4), 744-753 and Lecomte, E., et al., 2015, Molecular Therapy - Nucleic Acids, 4(10), e260). These stem from the plasmids used for rAAV production in cell culture and are packaged during vector production.

They are likely derived from the plasmid encoding the recombinant virus sequences and a gene of interest or sequence of interest to be introduced into the capsid used for obtaining virus like particles for gene therapy applications. However, these bacterial sequences are potentially immunogenic. Therefore, to be able to comply with the increasing demand for rAAV and safety, the present methods for production of the same must be improved or new methods must be developed. This is particularly true for repeated applications such as the directed tumor gene therapy. In directed tumor gene therapy, a broad spectrum of tumor diseases shall be treated with high doses of rAAV, different to the presently approved gene therapeutic therapies directed to orphan diseases.

AAV serves as a platform for the development of recombinant AAVs also identified here as AAV vectors. AAV are a member of the family P*arvoviridae* and belong to the genus *dependoparvovirus.* AAV are composed of a single stranded DNA genome of about 4.7 kB packaged into a non-enveloped protein capsid. Said capsid is of icosahedral shape and consists of 60 monomers. The monomers are composed of one of the viral capsid proteins VP1, VP2 or VP3. The 4.5 kB single-stranded regulatory or coding DNA genome (plus inverted terminal repeats, ITRs) is bearing two gene cassettes (*rep* and *cap*) encoding non-structural (Rep proteins and assembly activating protein) and structural proteins (VP1, VP2, VP3). The AAV genome is flanked by ITRs, which serve as packaging and replication signals. AAV is unique in being dependent of a helper virus like adenovirus for progeny production. In AAV vectors, viral open reading frames are replaced by foreign nucleic acids such as a transgene expression cassette or parts of the AAV nucleic acid sequence only. AAV vectors deliver a single stranded DNA encoding the sequence of interest or gene of interest flanked by the viral ITR structures. They are designed either in the natural genome conformation or as so-called self-complementary vector genomes. Vector genomes are packaged during vector production into natural occurring capsids (serotype/variants) or engineered capsids.

To produce rAAV for therapeutic applications in mammalian cell culture, the AAV genome is typically split into two plasmids, where one bears the two open reading frames of the AAV genome depleted of ITRs and the other plasmid carries the gene of interest flanked by ITRs, which mediate its encapsidation. A third plasmid provides further helper functions of the Adeno virus (Matsushita, T., et al., 1998, Gene Therapy, 5(7), 938-945 and Xiao, X., Li, J., & Samulski, R. J., 1998, Journal of Virology, 72(3), 2224-2232.). The two non-ITR bearing plasmids can be combined into one helper plasmid. Several AAV serotypes are characterized and their varying tropism can be exploited in therapy for tissue specific gene delivery. Pseudotyping (pseudopackaging), a strategy to change the tropism of the AAV vectors, further broadens their therapeutic reach. On the genetic level, sequences for tissue-specific peptides can also be incorporated into the capsid for altered tropism.

The problem of packaging of bacterial sequences may be circumvented using the minicircle technology of plasmid factory, Bielefeld, Germany (Schnödt, M., et al., 2016, Molecular Therapy - Nucleic Acids, 5(8), e355). The system is based on a bacterial plasmid including the GOI to be packaged into the capsids, flanked by ITRs and recombinase recognition sequences. During minicircle production, the parental plasmid is modified by a recombinase to a double stranded, covalently closed "minicircle" containing just the ITRs, a recombinase scar and the gene of interest. The minicircle then replaces the GOI plasmid during AAV vector production. Generation of minicircles from the helper plasmids is also possible. The described minicircle technology should overcome the problem of packaging bacterial sequences remaining in the DNA to be included into the capsids.

Another technique has been described for obtaining rAAVs essentially free of bacterial sequences. Scott, et.al., 2015 Human Vaccines & Immunotherapeutics, 11(8), 1972-1982 describe novel synthetic circular DNA vaccines, termed Doggybone DNA. This system is based on applying a rolling circle amplification of a DNA eventually obtaining double stranded DNA, which is used for the rolling circle amplification again, thereby providing exponential amplification. In a final step, a protelomerase resolves the amplification product to double-stranded fragments with covalently closed telomeric ends. The company Touchlight further develops and markets this technology. Therein, the use of two enzymes, namely the phi 29 DNA polymerase and a protelomerase to generate covalently closed, linear DNA constructs named Doggybone DNA or dbDNA is disclosed. Recently, the production of AAV particles using this technology has been described, see Karbowniczek, K., et. Al., 2017, Cell and Gene Therapy Insights, 3(9), 731-738.

This method is based on the covalently closed DNA construct being present as a circular DNA construct. Pending patent applications with respect to the described Doggybone system include WO 2018/033730 A1, WO 2016/122129 A1, WO 2016/034849 A1, WO 2012/017210 A1 and WO 2010/086626 A1.

Both techniques to produce rAAV essentially free of bacterial sequences known in the prior art-namely minicircle and Doggybone technology-provide synthetic DNA molecules different to the DNA molecules needed for rAAV genome amplification and packaging into the viral capsids, which are linear, single stranded molecules. Thus, production does not follow the natural route and in both cases might be suboptimal. Moreover, minicircle technology relies on DNA amplification in bacteria, where GC-rich repeat sequences, like ITRs, are known to be unstable. Further, Doggybone technology relies on exponential DNA amplification, possibly leading to the accumulation of amplification errors in the product, especially arising from GC-rich repeat sequences like ITRs.

With respect to the low production capacity and high production costs of rAAV, it is desirable to provide a single stranded DNA to be included into corresponding capsids including AAV capsids. Namely, including the single stranded gene of interest including an AAV genome or a GOI or SOI into the AAV capsids with a high number production of rAAV.

Rolling circle amplification is described in the art. Namely rolling circle amplification refers to a process of unidirectional nucleic acid amplification that can synthesize multiple copies of circular molecules of DNA or RNA, such as plasmids, including the genomes of bacteriophages, and the circular RNA genome of viroids. Typically, isothermal DNA amplification techniques are used for natural rolling circle amplification. In rolling circle amplification techniques, a polymerase continuously adds single nucleotides to a primer near to a circular template resulting in a long multimer containing single stranded DNA with tens to hundreds of copies of the circular template. Useful polymerases applicable in rolling circle amplification are the phi 29 DNA polymerase as an enzyme of the group of DNA polymerase obtainable from bacteriophages. Other samples include BST and the vent (exo-) DNA polymerase for DNA amplification as well as T7 polymerase for RNA amplification.

Often the rolling circle amplification technique is applied for determining and detecting targets with low abundance in a sample.

From the above, it is clear that there is a continuous demand for the production of nucleic acid fragments for the introduction into virus like particles or empty capsids in particular of AAV. Further, the provision of a method for the efficient production of rAAV is desired.

### Brief description of the present invention

In a first aspect, the present invention relates to a method for the *in vitro* generation of genetically engineered, linear, single-stranded nucleic acid (ssNA) fragments containing two viral inverted terminal repeat (ITR) sequences flanking a gene of interest (GOI), being essentially free of bacterial nucleic acid sequences, comprising the steps of:
a) Providing a bacterial nucleic acid plasmid containing a nucleic acid fragment containing the two viral ITR sequences and the GOI,
b) Enzymatic digestion of the plasmid with a first restriction enzyme (1) or a combination of at least two restriction enzymes (1) and (4) generating compatible ends with the double stranded nucleic acid fragment,
c) Isolation of the linear double stranded nucleic acid fragment containing the nucleic acid fragment with viral ITR sequences and GOI;
d) Recirculation of the isolated linear double stranded nucleic acid fragment of step c) to obtain a double stranded circular nucleic acid fragment joint at the compatible ends formed in step b);
e) Amplification of single stranded circular nucleic acid fragments comprising more than one of said nucleic acid fragment with the two viral ITR sequences and the GOI from the double stranded circular nucleic acid fragment of d) as the template whereby more than one nucleic acid fragments being covalently linked with each other are obtained;
f) Enzymatic digestion of the amplification product obtained in step e) with a second restriction enzyme (2) or at least two different restriction enzymes (2) and (3) generating compatible ends to obtain linear ssNA sequences comprising said nucleic acid fragment of the two viral ITR sequences and the GOI; and
g) Purification of said linear ssNA fragment obtained in step f) comprising the nucleic acid fragment of the two viral ITR sequences and the GOI.

In a further aspect, an isolated linear ssNA fragment obtainable by a method according to the present invention is provided.

Moreover, a method is disclosed for the production of recombinant virus particles, in particular recombinant AAV particles (rAAV), comprising the step of packaging of the linear ssNA comprising viral nucleic acid fragments according to the present invention i) into virus like particles, ii) a) by transfecting said nucleic acid in cells containing the additional genes and molecules for obtaining the recombinant virus particle, in particular rAAV, b) by transfecting said nucleic acid and additional plasmids containing helper functions, or c) by transfecting said nucleic acid and co-infecting with viruses providing helper functions.

Further, the rAAV obtainable by the method according to the present invention is described.

Finally, a plasmid comprising the following nucleic acid sequence elements:
a) A recognition sequence for a first restriction enzyme (1) generating ends, like sticky ends, being compatible to the generated ends of g);
b) Downstream a recognition sequence for a second restriction enzyme (2) generating ends, like sticky ends, being compatible to the generated ends of f) and said recognition sequence being different to the recognition sequence of a) and g);
c) Downstream a functional ITR sequence;
d) Downstream the GOI;
e) Downstream a functional ITR sequence being identical or different to the ITR sequence of c);
f) Downstream a recognition sequence for a third restriction enzyme (3) generating ends being compatible to the generated ends of b) but different to the recognition sequence of a) and g); whereby the third restriction enzyme (3) may be identical or different to the second restrictions enzyme (2) of b);
g) Downstream a recognition sequence for a fourth restriction enzyme (4) generating ends, being compatible to the ends generated in a); whereby said fourth restriction enzyme (4) may be identical or different to the first enzyme (1); whereby, if restriction enzymes are used, that can cut at different distances relative to the recognition site, the recognition sequences in a) and b) as well as in f) and g) can be at the same position and up to all recognition sequences can be identical;
is disclosed.

The plasmid, the rAAV and the other ssNAs described herein are particularly suitable in gene therapeutic methods, in particular, AAV based gene therapy. That is, the present invention discloses a method for treating individuals in need thereof, including monogenic disorders like Sickle cell anemia, Severe Combined Immunodeficiency (ADA-SCID / X-SCID), Cystic fibrosis, Hemophilia, Duchenne muscular dystrophy, Huntington's disease, Parkinson's, Hypercholesterolemia, Alpha-1 antitrypsin, Chronic granulomatous disease, Fanconi Anemia and Gaucher Disease, as well as polygenic disorders like Heart disease, Cancer, Diabetes type I and type II, Schizophrenia and Alzheimer's disease. Further possible treatments include communicable diseases, infections, virus diseases, neoplasms, immune system diseases, syndromes, digestive system diseases, gastrointestinal diseases, respiratory tract diseases, immunologic deficiency syndromes, sexually transmitted diseases, skin diseases, lung diseases, liver diseases, hematologic diseases, metabolic diseases, immunoproliferative disorders, leukemia, lymphatic diseases, endocrine system diseases, mental disorders, psychotic disorders, genital diseases, intestinal diseases, brain diseases, musculoskeletal diseases, colonic diseases, urologic diseases, neurologic manifestations, rectal diseases, congenital abnormalities, blood coagulation disorders, hemostatic disorders, hemorrhagic disorders, bone marrow diseases, prostatic diseases and neuromuscular diseases.

### Brief description of the drawings

Figure 1: Fig.1 is a scheme showing the different steps of the method according to the present invention.
Figure 2: Fig.2 shows the production and biological activity of the fragments according to the present invention (Fig. 2a and 2b) and of virus particles obtained according to the present invention.

### Detailed description of the present invention

The present inventors aim to provide a new method for the *in vitro* generation of genetically engineered, linear, single-stranded nucleic acid (ssNA) fragments which are useful for incorporation into viral vectors. The method according to the present invention is based on a rolling circle amplification resulting in ssNA fragments. That is, the present invention relates to a method for the *in vitro* generation of genetically engineered, linear, ssNA fragments containing two viral inverted terminal repeat (ITR) sequences flanking a gene of interest (GOI), being essentially free of bacterial nucleic acid sequences, comprising the steps of:
a) Providing a bacterial nucleic acid plasmid containing the nucleic acid fragment containing the two viral ITR sequences and the GOI,
b) Enzymatic digestion of the plasmid with a first restriction enzyme (1) or a combination of at least two restriction enzymes (1) and (4) generating compatible ends with the double stranded nucleic acid fragment,
c) Isolation of the linear double stranded nucleic acid fragments containing the nucleic acid fragment with viral ITR sequences and GOI;
d) Recirculation of the isolated linear double stranded nucleic acid fragments of step c) to obtain double stranded circular nucleic acid fragments joint at the compatible ends formed I step a);
e) Amplification of single stranded circular nucleic acid fragments comprising more than one nucleic acid fragments with the two viral ITR sequences and the GOI from the double stranded circular nucleic acid fragment of d) as the template whereby more than one nucleic acid fragments being covalently linked with each other;
f) Enzymatic digestion of the amplification product obtained in step e) with a second restriction enzyme (2) or at least two different restriction enzymes (2) and (3) generating compatible ends to obtain linear ssNA sequences comprising said nucleic acid fragment of the two viral ITR sequences and the GOI; and
g) Purification of said linear ssNA fragment obtained in step f) comprising the nucleic acid fragment of the two viral ITR sequences and the GOI.

That is, the present inventors recognized that applying the rolling circle amplification to a specifically designed bacterial plasmid with the specific functions as described allows to obtain linear, ssNA fragments containing functional viral ITR sequences and a gene of interest essentially free, in particular, free of any bacterial sequences increasing safety and apathogenicity of resulting viral particles. The linear ssNA fragments retain their biological function and are suitable for the production of recombinant virus particles In particular, *in vitro* production of viral particles from virus like particles and production of rAAV's *in vitro* with suitable load of nucleic acid fragments is possible. Said load include the linear ssNA fragment according to the present invention. Namely, the fragment according to the present invention obtainable by the method according to the present invention represents an optimal substrate for *in vitro* packaging, thus, obtaining recombinant virus particles, like rAAV's. Using the linear ssNA fragments according to the present invention allows to reduce the production process and the costs of the production as well as increasing safety aspects etc.

As used herein, the term "genetically engineered" refers to an artificially modified nucleic acid molecule obtained by recombinant nucleic acid technology.

The term "nucleic acid" includes DNA, RNA as well as variants thereof such as siRNA, miRNA as well as other known nucleic acid variants like PNA, lock nucleic acid (LNA) as well as glycol nucleic acid (GNA) and triose nucleic acid (TNA).

The term "ITR sequence(s)" refers to the nucleic acid sequence serving as the viral origin of replication being also required for packaging. That is, a functional ITR includes the packaging and replication signals. Typically, the ITR are present at the ends of the viral genome, like in the Adeno-associated virus genome.
According to the present invention, when identifying the ITR sequences, the ITR sequences are based on viral sequences as described in the art or fragments thereof whereby said fragments according to the present invention are ITR fragments having the same major functionality as the complete ITR sequences, namely, allowing packaging and replication. For example, minimal ITR sequences are described by Zhou, Q., et. al., 2017, Scientific Reports, 7(1), 5432).

The term "substantially free of bacterial nucleic acid sequences", as used herein, identifies that the nucleic acid fragments according to the present invention do not contain any bacterial nucleic acid sequences, namely, nucleic acid sequences stemming from the bacterial plasmid or contain only very few bacterial nucleic acid bases, like at most 40, 30, 25, 20, 15, 12, 10, 9, 8, 7, 6, 5, 4, 3, 2 or 1 nucleic acid bases.

In an embodiment of the present invention, the genetically engineered, linear, ssNA fragment according to the present invention containing the two viral inverted terminally repeat sequences flanking a gene of interest is free of bacterial nucleic acid sequences. Thus, it is possible to increase safety of the virus particles containing the same as well as increase the feasibility for drug approval.

In particular, the full absence of bacterial sequences increases safety in the production of rAAV's by *in vitro* packaging, in particular, these recombinant virus particles, like rAAVs are useful in gene therapy.

As used herein, the term "compatible ends" refers to five prime and three prime ends of double stranded nucleic acid fragments wherein at least one nucleic acid overhangs the double stranded fragment, namely, being an unpaired nucleic acid of a single strand, thus, representing sticky ends. These compatible ends are generated by restriction enzymes. With a complementary base pair or complementary base pairs, these overhangs or sticky ends represent the compatible ends, capable of forming double stranded nucleic acids.

Further, the compatible ends also include blunt ends, namely, double stranded fragments wherein both strands terminate in a base pair.

The term" "restriction enzyme" includes suitable enzymes digesting double stranded nucleic acid molecules, which can be conventional restriction enzymes known in the art or designer restriction enzymes which can be based on CRISPR enzymes, TALENs or zinc finger nucleases and homing endonucleases.

The term "amplification" refers to the duplication or templated synthesis of nucleic acids. Amplification is possible by various methods including polymerase chain reaction, rolling circle amplification and (loop mediated) isothermal amplification.

The term "complementary sequence" or "complementary" refers to a nucleic acid sequence of nucleic acid molecules that can form a double-stranded structure by matching base pairs. That is, the complementary sequences form a double-stranded nucleic acid structure matching with the second ssNA sequence. For example, in case of compatible ends or sticky ends obtained by restriction enzymes, the complementary sequence is the nucleic acid sequence of base pairs forming the double-stranded structure by matching of the base pairs.

The terms "vector particle", or "vector" or "virus particle" identify a viral capsid containing transcriptionally active nucleic acid, like DNA, in particular, a viral, transcriptionally active nucleic acid, like DNA.

The terms "particle" or "virus like particle (VLP)" identify a capsid without nucleic acid, like DNA, or containing transcriptionally inactive nucleic acid, like DNA, in particular, non-viral transcriptionally inactive nucleic acid, like DNA.

The term "AAV" refers to the Adeno-associated virus or the derivatives thereof including recombinant AAV vector particles wherein the term "AAV wildtype particle" designates the Adeno-associated virus capsid as it occurs in nature without DNA or containing DNA. The term "recombinant AAV" or "recombinant vector particle" is an AAV wherein the genome of the virus is substituted with a vector genome, namely, a foreign DNA to be introduced into the cell. Said recombinant AAV is also referred to as "rAAV". The term "AAV" or the respective recombinant AAV vector particle and AAV wildtype particle include at least 13 different serotypes known in the art. The AAV of human serotype 2 is also mentioned as AAV 2 or AAV 2 particle or AAV 2 vector particle. AAV 2 is a preferred embodiment. The term AAV its variations also refers to pseudo-typed, chimeric or rationally modified capsids.

The term *"in vitro"* as used herein refer to a cell-free method of obtaining the ssNA according to the present invention. No incubation with, or transformation of living biological material etc. is required starting from the plasmid to obtain the ssNA according to the present invention.

The vector genome, like the foreign DNA to be introduced into the cells, present in the recombinant vector particle or recombinant AAV, may be the natural virus genome, like the natural AAV genome or, in another embodiment, a foreign DNA containing a gene of interest (GOI) or sequences of interest (SOI) to be introduced into the cell.

Typical examples of a gene of interest or a sequence of interest to be introduced into the cell, for example in gene therapy, include genes coding for: adhesion molecules, antigens, antisense DNA, cell cycle arrestor, drug resistance marker, cytokines, enzyme replacements, growth factors, hormones, markers, oncogene regulators, porins, ion channels, transporters, receptors, replication inhibitors, ribozymes, siRNA, suicide genes, transcription factors, tumor suppressors antibody derived sequence and antibody mimetics such as DARPins, Affibodies, Anticalins, Adenectins, Affilins, and Nanobodies.

In an embodiment of the present invention, the method for the *in vitro* generation of linear single stranded viral nucleic acid fragments is a method using a restriction enzyme (1) and/or (4) generating sticky ends. These restriction enzymes (1) and/or (4) include type II enzymes, like Pst I, type IIs endonucleases and designer endonucleases.

In another embodiment of the method according to the present invention, the restriction enzyme (2) and/or (3) being different to the restriction enzymes (1) and (4) is a blunt-end cutting restriction enzyme, e.g. of type II, like Pvull, or type IIs or a designer endonuclease. Alternatively, the second restriction enzyme may be an enzyme generating sticky ends.

In an embodiment, restriction enzymes (1) and (4) may be used to digest the double-stranded nucleic acid into fragments with compatible ends. The same is possible for the restriction enzymes (2) and (3).

In an embodiment of the present invention, the method for the *in vitro* generation of linear single stranded genetically engineered viral nucleic acid fragments is a method wherein the plasmid containing viral nucleic acid fragments containing the sequence elements:
a) A recognition sequence for a restriction enzyme (1) generating ends, like sticky ends, being compatible to the generated ends of g);
b) Downstream a recognition sequence for a restriction enzyme (2) generating ends, like sticky ends, being compatible to the generated ends of f), and said recognition sequence being different to the recognition sequence of a) and g);
c) Downstream a functional ITR sequence;
d) Downstream the GOI;
e) Downstream a functional ITR sequence being identical or different to the functional ITR sequence of c);
f) Downstream a recognition sequence for a restriction enzyme (3) generating ends being compatible generated ends of b) but different to the ends of a) and g), whereby the restriction enzyme (3) may be identical or different to the restriction enzyme (2) of b);
g) Downstream a recognition sequence for a restriction enzyme (4) generating ends, being compatible to the ends generated in a.) whereby said restriction enzyme (4) may be identical or different to the restriction enzyme (1); whereby if restriction enzymes are used, that can cut at different distances relative to the recognition site, the recognition sequences in a) and b) as well as in f) and g) can be at the same position and up to all recognition sequences can be identical.

In another embodiment, the recirculation step of the nucleic acid fragment in step b) may be conducted by a ligase, in particular, a T4 ligase. The skilled person is well aware of suitable enzymes and conditions for recirculation of the double-stranded nucleic acid fragment at the compatible ends obtained after digestion with the restriction enzymes.

In another embodiment, the method includes the step of providing an oligonucleotide to the recirculated isolated nucleic acid obtained in step d) and hybridizing said oligonucleotide specifically to a single nucleic acid strand of said nucleic acid. Alternatively, the nucleic acid obtained in step d) contains a nicking site allowing for nicking specifically one strand of said nucleic acid. The skilled person is well aware of suitable nicking enzymes, like nicking endonuclease, e. g. Nt.BspQI.

Another embodiment of the present invention relates to a method wherein hybridization of the oligonucleotide is conducted by heat denaturation of nucleic acid obtained in step d) and subsequent cooling of the reaction mixture.

In another embodiment, the method includes the steps of i.) isolation of the double stranded nucleic acid obtained in step d) and/or ii.) purification of the linear single-stranded nucleic acid in step h), is conducted by electrophoreses or ion exchange chromatography or silica adsorption chromatography.

In another embodiment, the present invention relates to a method wherein the amplification step e) is performed enzymatically by a polymerase. The polymerase is in a preferred embodiment the phi29 polymerase. The amplification step may be an isothermal reaction, which avoids thermal cycling.

That is, a preferred embodiment relates to a method wherein the amplification step e) is performed in the presence of the phi29 polymerase as isothermal nucleic acid amplification, in particular, isothermal DNA amplification.

Further, the present invention relates to a method wherein in step e) the product obtained by amplification of the single stranded circular DNA fragment is heat denaturated and cooled to facilitate hybridization of adjacent inverted terminal repeat and restriction site sequences to form a double-stranded hairpin containing a functional restriction site. This double-stranded hairpin containing the functional restriction site is particularly useful for further processing.

In a further embodiment, an isolated linear ssNA comprising viral nucleic acid fragments obtainable by a method according to the present invention is provided. Said isolated linear single-stranded nucleic acid, in particular, a DNA genome, is useful for the preparation of recombinant viruses, in particular, rAAV, for medical applications, in particular, gene therapy.

Further, the present invention provides a method for the production of recombinant virus particle, in particular, recombinant AAV particle (rAAV) comprising the step of packaging of the linear ssNA comprising viral nucleic acid fragments according to the present invention, or the step of packaging of the linear ssNA fragment comprising viral nucleic acid fragments, like DNA fragments, obtainable by a method according to the present invention into virus like particle, i) by transfecting said ssNA in cells containing the additional genes and molecule for obtaining the recombinant virus particle, in particular, rAAV, ii) by transfecting said ssNA and additional plasmids containing helper functions or iii) by transfecting said ssNA and co-infecting with viruses providing helper functions. Further, recombinant virus particles, like rAAV, can be produced cell-free, i.e. without any eukaryotic cells, by introducing the ssNA according to the present invention into VLP, like AAV capsids, produced in eukaryotic cells including plant cells, prokaryotic cells, or archeaea, like prokaryotic cells, or by chemical synthesis.

The method for the production of recombinant virus particle, in particular, rAAV, can be performed by known means. For example, the isolated genetically engineered linear ssNA fragment according to the present invention or obtainable by a method according to the present invention is included into a virus-like particle. It is particularly preferred that the method is an *in vitro* method for obtaining rAAV's useful in gene therapy. This *in vitro* generation and *in vitro* packaging increase the safety of the product obtained. For example, viral capsids not containing any DNA may be produced in suitable cells and the nucleic acid fragment according to the present invention is introduced into said empty viral capsids at a later time point. Suitable cells to produce empty capsids include eukaryotic cells, prokaryotic cells, and other cells, including human kidney derived cells, other human cells, hamster ovary cells, other mammalian cells, yeast, insect cells, archaea and bacterial cells, algae cells and other plant cells. In an embodiment of the method according to the present invention for the production of recombinant virus particle, said method is a cell free production of viruses, in particular, AAV. In another aspect, the recombinant AAV itself is disclosed obtainable by a method according to the present invention for producing recombinant virus particle. Said rAAV contain the isolated genetically engineered linear ssNA according to the present invention.

Further, the present invention provides a plasmid comprising the following nucleic acid sequence elements:
a) A recognition sequence for a first restriction enzyme (1) generating ends, like sticky ends, being compatible to the generated ends of g);
b) Downstream a recognition sequence for a second restriction enzyme (2) generating ends, like sticky ends, being compatible to the generated end of f) and said recognition sequence being different to the recognition sequence of a) and g);
c) Downstream a functional ITR sequence;
d) Downstream the GOI;
e) Downstream a functional ITR sequence being identical or different to the functional ITR sequence of c);
f) Downstream a recognition sequence for a third restriction enzyme (3) generating ends generating ends being compatible to the generated ends in b) but different to the recognition sequence of a) and g); whereby the third restriction enzyme (3) may be identical or different to the second restrictions enzyme (2) of b);
g) Downstream a recognition sequence for a fourth restriction enzyme (4) generating ends, being compatible to the generated in a) whereby said fourth restriction enzyme (4) may be identical or different to the first enzyme (1) whereby if restriction enzymes are used, that can cut at different distances relative to the recognition site, the recognition sequences in a) and b) as well as f) and g) can be at the same position and up to all recognition sequences can be identical.

The plasmid may contain additionally a sequence for a nicking enzyme, like a nicking endonuclease. Said recognition sequence may be preferably present in the GOI sequence. The same is true for the plasmid described in the method according to the present invention.

In a further embodiment the plasmid contains the recognition sequence of the restriction enzyme (2) and (3) being located to cut directly upstream or downstream, respectively, of the first or last respective, nucleotide of the ITR sequences.

The structure of the plasmid according to the present invention allows to generate a linear ssNA fragment containing no or being essentially free of bacterial nucleic acid sequences. In particular, the plasmid according to the present invention is particularly suitable for the *in vitro* generation of said ssNA containing two viral ITR sequences flanking a GOI according to the present invention.

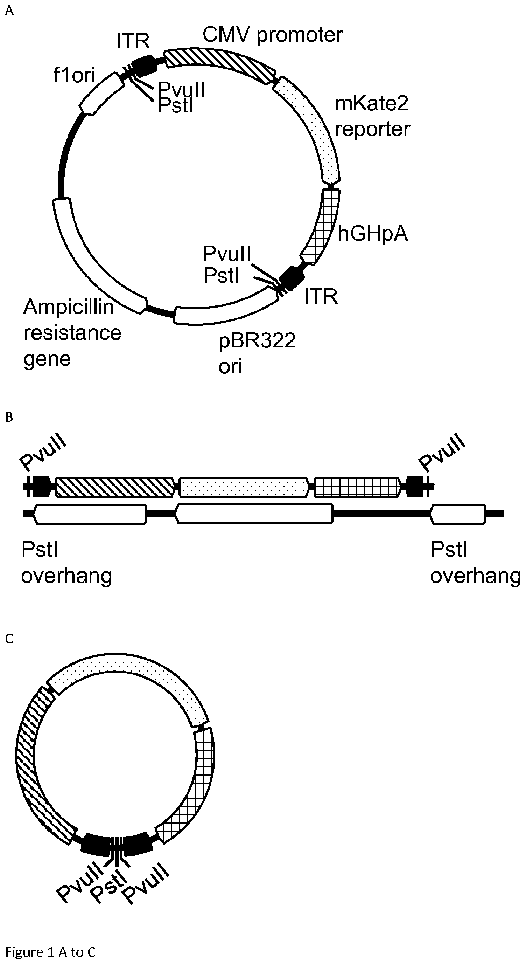

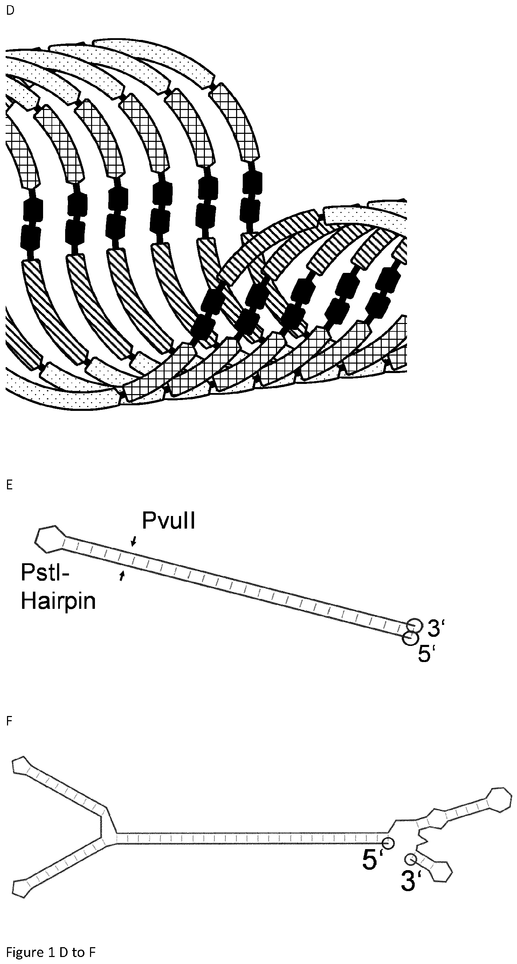

The invention will be described further referring to figure 1.

Figure 1 provides an overview of the vector plasmid and the various steps of the method for the *in vitro* generation of genetically engineered, linear, ssNA fragments containing two viral inverted terminal repeat (ITR) sequences flanking a gene of interest (GOI) according to the present invention. Scheme (A) depicts the vector plasmid useful for synthetic genome production. It contains wild type ITRs of AAV 2 shown in black. This type of ITR is present in most of AAV vector plasmids presently used. The two ITR sequences are flanking the GOI in the present case a promotor (hatched), followed by the coding sequence of a fluorescence reporter protein (dotted), further followed by a polyadenylation signal sequence (gridded). Recognition sites for restriction enzymes Pstl and Pvull are given as an ITR prefix or suffix for excision and recirculation as well as rolling circle product resolution.

In the present case, the ITR prefix contains a Pstl site, thus, allowing enzymatic digestion according to step b) of the method according to the present invention. Scheme (B) shows the result of the Pstl digestion, which generates two fragments, of which only the fragment containing the GOI is processed further.

After isolation of the fragment containing the GOI, recirculation is achieved with the sticky ends generated by Pstl, thus, obtaining a circular double stranded template for rolling circle amplification as shown in scheme (C) of figure 1. That is, digestion with the first restriction enzyme (1) and forth restriction enzyme (4), here both Pstl, yields a backbone fragment composed of bacterial sequences which are discarded and the GOI fragment. The GOI fragment is recirculated in step d) of the method according to the present invention by self ligation to yield a double-stranded nucleic acid fragment shown in scheme (C). This template contains two adjacent ITR's in a head to head reverse complementary configuration. One Pstl site and the two Pvull sites are preserved. The two Pvull sites correspond to the restriction sites for the second restriction enzyme (2) and third restriction enzyme (3).

Rolling circle amplification of step e) of the circular template depicted in scheme (C) with a single primer produces single stranded high molecular weight multimers of the AAV synthetic genome with a single polarity shown in (D). The multimers are present as covalently bonded monomers of the ssNA containing the two viral ITR sequences flanking the GOI.

The head to head nature of the amplicon leads probably to hairpin formation at the recirculation scar with a double stranded Pvull site that is used to release the genome monomer. Scheme (E) illustrates a cutout of this hairpin structure shown together with the Pstl hairpin and the Pvull restriction site. Even if the two ITR's form a Y-shaped structure as shown in scheme (F), a short restriction enzyme recognition side hairpin probably forms between two ITR's and suffices for cleavage. It is noted that the secondary structure of the DNA obtained after the rolling circle amplification suitable for cutting by the restrictions enzyme as described is distinct from the Y-shape structure proposed for ITR's *in vivo.* The enzymatic digestion of the amplification product results in single stranded linear nucleic acid fragments.

Thus, the method according to the present invention is different to the methods described so far. The linear ssNA fragment obtained after the second digestion is purified further, e. g. by way of ionic exchange chromatography. Thus, it is possible to obtain a linear ssNA fragment which is ready to be packaged into virus like particles or capsids. The invention will be described further by way of examples. It is clear that the examples do not restrict the subject matter of the present invention.

### Example:

A plasmid is provided as shown in figure 1 containing: From 5' to 3': a Pstl restriction site followed by a Pvull restriction site, followed directly by a functional AAV2 ITR sequence as present on common AAV vector plasmids, further followed by a cytomegalovirus promoter, followed by the coding sequence for fluorescence reporter protein mKate2, followed by the polyadenylation signal of the human growth hormone as shown in SEQ ID No. 1. The sequence is followed further by an inverse complement of the 5' ITR sequence harboring a deletion of ITR nucleobases 5'-TTT-GCCCGGGC (SEQ ID No. 2), and by the Pvull and Pstl restriction sites. Further 3' sequence elements are a pBR322 origin of replication, an ampicillin resistance gene and a f1 origin of replication.

In a first step, enzymatic digestion is conducted using Pstl as follows: 10 µg of the before described plasmid are digested with 2 µl of commercially available Pstl-HF (New England Biolabs) per the manufacturer's instructions an a final volume of 50 µl and in the buffer suggested by the manufacturer for at least 2 hours at 37 °C.

The fragments obtained are separated by electrophoresis, thus isolating the double stranded nucleic acid fragment containing the nucleic acid fragment with viral ITR sequences and the GOI. Separation is performed in an agarose gel in Tris-Acetate-EDTA buffer. Gel extraction is performed with a commercially available kit (NucleoSpin Gel and PCR Clean-up, Macherey-Nagel). The isolated fragment is recirculated using commercially available T4 ligase (Thermo Scientific). A 200 µl reaction mixture is prepared containing 300 ng of isolated fragment, 10 µl of T4 ligase and commercially available phi29 polymerase reaction buffer (New England Biolabs) supplemented to a final concentration of 1 mM ATP and 10 mM DTT. The mixture is incubated for 1 hour at room temperature.

The circular double stranded nucleic acid fragments thus obtained joint at the compatible ends formed by digestion with Pstl are hybridized with a primer 5'-GGCGGAGTTGTTACGACA (SEQ ID. No. 3) by adding the primer to a final concentration of 180 nM and incubating the reaction mixture at 95 °C for two minutes and cooling it back to room temperature. A polymerase with high processability and strong strand displacement activity, here phi29 polymerase, is added to the circular DNA fragments to start the amplification. For amplification, the mixture is brought to a final volume of 300 µl including 0.7 mM dNTPs, 0.3 mg/ml BSA and 5 µl of commercial phi29 polymerase (New England Biolabs). By rolling circle amplification at 30 °C for two days, single stranded multimers of the DNA fragments are obtained whereby the flanking ITR's are present as single strands covalently bound with each other in short distance. Next, by heating and slow cooling, a DNA hairpin is formed with a recognition site for the second restriction enzyme, here Pvull. The multimeric product obtained after the rolling circle amplification is digested with Pvull by bringing the reaction mixture to a final volume of 600 µl, including 15 µl of Pvull-HF restriction enzyme (New England Biolabs) and the buffer recommended by the manufacturer. The mixture is incubated at 37 °C for 300 minutes to obtain a linear ssNA fragment containing the two ITR sequences flanking the GOI. These fragments are purified by a silica-based PCR cleanup kit (Macherey Nagel).

The fragments obtained by the method from the example are subsequently analyzed by denaturing polyacrylamide gel electrophoreses. Samples are denatured by heating in 8 M urea solution and separated on an 8 % acrylamide gel containing 8 M urea in Tris-borate EDTA buffer run at 6 V/cm for six hours. RotiSafe gel stain (Carl Roth) is used to visualize the fragments on a blue light table.

The biological activity of the linear ssNA fragment containing the two ITR sequences flanking the fluorescence reporter gene in terms of transfection and virus production is tested in cell culture experiments. Adherent HEK293 cells are grown in commercial DMEM culture media containing 10 % fetal calf serum. Cells are seeded at 52,000 cells per cm² culture surface one day before transfection experiments. The next day, cells are transfected by the calcium phosphate transfection method. For transfection of 3 x 10⁶ seeded cells, a total of 15 µg DNA is diluted in 0.25 ml of 0.3 M CaCl₂ solution. The DNA solution is added dropwise to an equal volume of HEPES-buffered saline containing disodium phosphate (50 mM HEPES, 1.5 mM disodium phosphate, 280 mM sodium chloride, pH 7.05). The resulting mixture is slowly and dropwise added to the cells. Either only the DNA fragment obtained by the method described in the example is used for transfection, or a molar 1:1:1 ratio of the fragment from the example, a plasmid encoding the adeno-associated virus capsid proteins and its helper-genes (pRepCap, e.g. according to SEQ ID No. 5) and an adeno-virus helper plasmid (pHelper, Agilent technologies AAV helper free system) to produce virus particles. pRepCap and pHelper are well-known to the prior art.

Further analysis of the transfected cells is performed after three days of subsequent cultivation, either by fluorescence imaging using a fluorescence microscope with a filter set suitable for the fluorescence reporter mKate2, or by quantitative PCR to determine the titer of produced virus particles. Samples for quantitative PCR are prepared by lysis of transfected cells. For this, cells are harvested by scraping and pelleted by centrifugation. The pellet is resuspended in Tris-buffered saline and subjected to three freeze-thaw cycles. Cellular debris is pelleted by centrifugation and the supernatant containing the virus particles is collected and filtered sterile. A 2.5 µl sample of this lysate is incubated with an equal volume of DNasel (New England Biolabs) in a 25 µl reaction volume as described by the manufacturer, to digest all DNA not packaged in virus capsids. The mixture is then diluted 5-fold with distilled water and 5 µl of the dilution is used in a 20 µl qPCR reaction with primers 5'-GGGACTTTCCTACTTGGCA (SEQ ID. No. 4) and 5'- GGCGGAGTTGTTACGACA (SEQ ID. No. 3).

### Results:

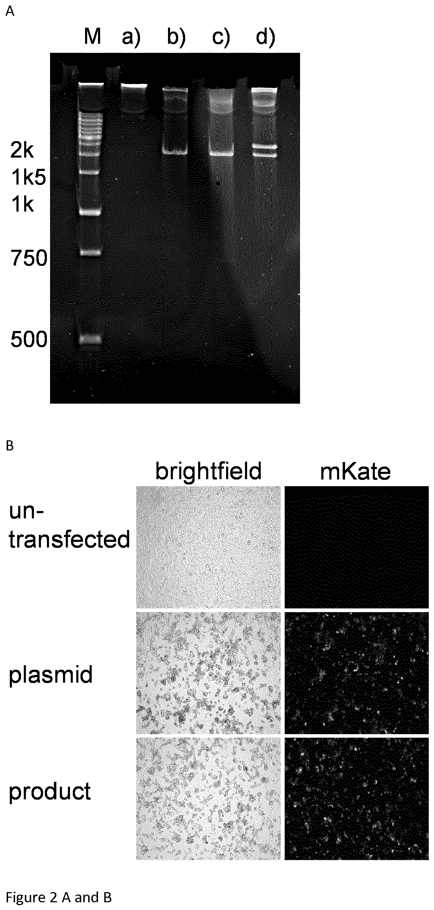

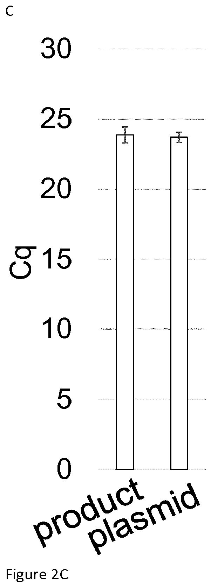

The results from the example will be described referring to figure 2.

Scheme (A) of figure 2 shows a denaturing PAGE of the resulting DNA fragments and process intermediate steps of the example process to generate linear, ssNA fragments containing two viral inverted terminal repeat sequences flanking a gene of interest according to the present invention. Lane d) of the gel shows the Pstl digestion of the plasmid according to step b) of the current invention. Two bands are clearly visible with the smaller of which being the ITR containing fragment with a size of 2.2 kb and the larger fragment being the plasmid backbone. Lane a) shows the result of the rolling circle amplification present as high molecular weight fragments unable to migrate into the gel. Lane b) shows the sample of lane a), but digested with Pvull, leading to release of the desired single stranded fragment. Lane c) shows the final product purified by a silica-based purification method.

The single stranded linear nucleic acid fragment according to the present invention obtained above demonstrates biological activity in HEK293 cells. Scheme (B) of figure 2 shows the result of a transfection experiment where HEK293 were either not transfected as a control (upper two panels), transfected with the plasmid according to the present invention (middle two panels), or transfected with an equal molar amount of the linear, single stranded fragments according to the present invention (lower two panels). The production of the reporter protein mKate2 was determined in all three cases by fluorescence imaging (panels on the right). As evident from the presented images, fluorescence emission as a measure of recombinant protein production is comparable between the two transfections, demonstrating that the biological activity of the linear single stranded fragment in terms of transfectability and protein expression equals that of its progenitor plasmid, while at the same time avoiding the transfection of prokaryotic DNA.

To identify further whether the linear ssNA fragments containing the two viral ITR sequences flanking the gene of interest are suitable for production of virus particles, a test was conducted based on a three plasmid system as described by Matsushita T. et al, and by Xiao X. et al, see above. The progenitor plasmid according to the present invention was used as reference. After a three-day production in HEK293 cells, the amount of DNasel resistant particles was determined as a measure of qPCR quantification cycle (Cq). As shown in scheme (C) of figure 2, comparable amounts are present in the control as well as in the virus sample produced with the ssNA fragment according to the present invention. That is, the biological functions of the nucleic acid according to the present invention, namely, competence in virus particle production and gene expression, as well as transfectability are similar to plasmids currently used to produce recombinant AAV, while at the same time avoiding the transfection of prokaryotic DNA.

## Claims

1. Method for the *in vitro* generation of genetically engineered, linear, single-stranded nucleic acid (ssNA) fragments containing two viral inverted terminal repeat (ITR) sequences flanking a gene of interest (GOI), being essentially free of bacterial nucleic acid sequences, comprising the steps of:
a) Providing a bacterial nucleic acid plasmid containing a nucleic acid fragment containing the two viral ITR sequences and the GOI,
b) Enzymatic digestion of the plasmid with a first restriction enzyme (1) or a combination of at least two restriction enzymes (1) and (4) generating compatible ends with the double stranded nucleic acid fragment,
c) Isolation of the linear double stranded nucleic acid fragments containing the nucleic acid fragment with viral ITR sequences and GOI;
d) Recirculation of the isolated linear double stranded nucleic acid fragments of step c.) to obtain a double stranded circular nucleic acid fragments joint at the compatible ends formed in step b);
e) Amplification of single stranded circular nucleic acid fragments comprising more than one of said nucleic acid fragment with the two viral ITR sequences and the GOI from the double stranded circular nucleic acid fragment of d) as the template, whereby more than one nucleic acid fragments being covalently linked with each other are obtained;
f) Enzymatic digestion of the amplification product obtained in step e) with a second restriction enzyme (2) or at least two different restriction enzymes (2) and (3) generating compatible ends to obtain linear ssNA sequences comprising said nucleic acid fragment of the two viral ITR sequences and the GOI; and
g) Purification of said linear ssNA fragment obtained in step f) comprising the nucleic acid fragment of the two viral ITR sequences and the GOI.

2. The method for the *in vitro* generation of linear single stranded viral nucleic acid fragments according to claim 1 wherein the first restriction enzyme (1) and fourth restriction enzyme (4) is a sticky end generating enzyme, like Pstl.

3. The method for the *in vitro* generation of linear single stranded viral nucleic acid fragments according to claim 1 or 2 wherein the second restriction enzyme (2) and third restriction enzyme (3) is a blunt end cutting restriction enzyme, like Pvull.

4. The method for the *in vitro* generation of linear single stranded genetically engineered viral nucleic acid fragments according to any one of claims 1 to 3, wherein the plasmid containing viral nucleic acid fragments containing the sequence elements:
a) A recognition sequence for a restriction enzyme (1) generating ends, like sticky ends, being compatible to the generated endsof g);
b) Downstream a recognition sequence for a restriction enzyme (2) generating ends, like sticky ends, being compatible to the generated ends of f), and said recognition sequence being different to the recognition sequence of a) and g);
c) Downstream a functional ITR sequence;
d) Downstream the GOI;
e) Downstream a functional ITR sequence being identical or different to the functional ITR sequence of c);
f) Downstream a recognition sequence for a restriction enzyme (3) generating ends being compatible generated ends of b) but different to the ends of a) and g), whereby the restriction enzyme (3) may be identical or different to the restriction enzyme (2) of b);
g) Downstream a recognition sequence for a restriction enzyme (4) generating ends, being compatible to the ends generated in a.) whereby said restriction enzyme (4) may be identical or different to the restriction enzyme (1) ;whereby if restriction enzymes are used, that can cut at different distances relative to the recognition site, the recognition sequences in a) and b) as well as in f) and g) can be at the same position and up to all recognition sequences can be identical.

5. The method according to any one of the preceding claims wherein i.) isolation of the double stranded nucleic acid fragment obtained in step d) containing the viral nucleic acid fragment and/or ii.) purification of the linear ssNA fragment comprising viral nucleic acid fragments in step g), is conducted by electrophoreses or ion exchange chromatography or silica adsorption chromatography.

6. The method according to any one of the preceding claims wherein recirculation of the nucleic acid fragment in step d) is conducted by a ligase, in particular, a T4 ligase.

7. The method according to any one of the preceding claims wherein the amplification step e) is performed enzymatically by a polymerase; preferably, the polymerase is the phi29 polymerase; and/or preferably the amplification is an isothermal reaction.

8. A method according to any one of the preceding claims wherein hybridization of an oligonucleotide for amplification in step e) is conducted by heat denaturation of said circular double stranded nucleic acid fragment and subsequent cooling of the reaction mixture.

9. The method according to any one of the preceding claims wherein in step e) the amplification product obtained by amplification of the single stranded circular nucleic acid fragment, in particular, the DNA fragment, is heat denatured and cooled to allow hybridization of adjacent inverted terminal repeat and restriction site sequences to form a double stranded hairpin containing a functional restriction site.

10. An isolated linear ssNA fragment comprising viral nucleic acid fragments obtainable by a method according to anyone of claims 1 to 9.

11. A method for the production of recombinant virus particle, in particular, recombinant AAV particle (rAAV), comprising the step of packaging of the linear ssNA fragment comprising viral nucleic acid fragments according to claim 10 or the linear ssNA fragment comprising viral nucleic acid fragments obtainable by a method according to any one of claims 1 to 9 into virus like particle, i) by transfecting said ssNA in cells containing the additional genes and molecules for obtaining the recombinant virus particle, in particular, rAAV, ii) by transfecting said ssNA and additional plasmids containing helper functions, iii) by transfecting said ssNA and co-infecting with viruses providing helper functions or iv) cell-free by introducing the ssNA according to the present invention into VLP, like AAV capsids, produced in cells, like prokaryotic cells, or by chemical synthesis.

12. The method according to claim 11 for cell free production of rAAV.

13. rAAV obtainable by a method according to anyone of claims 11 to 12.

14. A plasmid comprising the following nucleic acid sequence elements:
a) A recognition sequence for a first restriction enzyme (1) generating ends, like sticky ends, being compatible to the generated endsof g);
b) Downstream a recognition sequence for a second restriction enzyme (2) generating ends, like sticky ends, being compatible to the generated end of f) and said recognition sequence being different to the recognition sequence of a) and g);
c) Downstream a functional ITR sequence;
d) Downstream the GOI;
e) Downstream a functional ITR sequence being identical or different to the functional ITR sequence of c);
f) Downstream a recognition sequence for a third restriction enzyme (3) generating ends generating ends being compatible to the generated ends in b) but different to the recognition sequence of a) and g); whereby the third restriction enzyme (3) may be identical or different to the second restrictions enzyme (2) of b);
g) Downstream a recognition sequence for a fourth restriction enzyme (4) generating ends, being compatilbe to the generated in a) whereby said fourth restriction enzyme (4) may be identical or different to the first enzyme (1) whereby if restriction enzymes are used, that can cut at different distances relative to the recognition site, the recognition sequences in a) and b) as well as f) and g) can be at the same position and up to all recognition sequences can be identical.

15. The plasmid according to claim 14 wherein the recognition sequence of the restriction enzyme (2) and (3) is located to cut directly upstream to the first nucleotide of the inverted terminal repeat sequence.


SEQUENCE LISTING

<110>  Universitaet Bielefeld

<120>  Method for the production of rAAV and method for the in vitro
       generation of genetically engineered, linear, single-stranded
       nucleic acid fragments containing ITR sequences flanking a gene
       of interest

<130>  6209-001 EP-1

<160>  7

<170>  PatentIn version 3.5

<210>  1
<211>  4792
<212>  DNA
<213>  artificial

<220>
<223>  genetically engineered plasmid of the example



<220>
<221>  rep_origin
<222>  (1)..(422)
<223>  in combination with rep_origin 4595 to 4792

<220>
<221>  misc_feature
<222>  (469)..(609)
<223>  ITR

<220>
<221>  promoter
<222>  (659)..(1312)

<220>
<221>  CDS
<222>  (1319)..(2014)
<223>  mKate2

<220>
<221>  polyA_site
<222>  (2023)..(2501)
<223>  complement

<220>
<221>  polyA_signal
<222>  (2105)..(2110)

<220>
<221>  misc_feature
<222>  (2534)..(2663)
<223>  ITR complement

<220>
<221>  rep_origin
<222>  (2755)..(3061)

<220>
<221>  promoter

<222>  (3510)..(3538)

<220>
<221>  CDS
<222>  (3580)..(4440)
<223>  AmpR

<220>
<221>  rep_origin
<222>  (4595)..(4792)
<223>  in combination with rep_origin of 1 to 422

<400>  1
agaactctgt agcaccgcct acatacctcg ctctgctaat cctgttacca gtggctgctg      60

ccagtggcga taagtcgtgt cttaccgggt tggactcaag acgatagtta ccggataagg     120

cgcagcggtc gggctgaacg gggggttcgt gcacacagcc cagcttggag cgaacgacct     180

acaccgaact gagataccta cagcgtgagc tatgagaaag cgccacgctt cccgaaggga     240

gaaaggcgga caggtatccg gtaagcggca gggtcggaac aggagagcgc acgagggagc     300

ttccaggggg aaacgcctgg tatctttata gtcctgtcgg gtttcgccac ctctgacttg     360

agcgtcgatt tttgtgatgc tcgtcagggg ggcggagcct atggaaaaac gccagcaacg     420

cggccttttt acggttcctg gccttttgct ggccttttgc tcacatgtcc tgcaggcagc     480

tgcgcgctcg ctcgctcact gaggccgccc gggcaaagcc cgggcgtcgg gcgacctttg     540

gtcgcccggc ctcagtgagc gagcgagcgc gcagagaggg agtggccaac tccatcacta     600

ggggttcctg cggccgccta tcgagaccgg cgccgcgaat tcgcggccgc ttctagagcg     660

atgtacgggc cagatatacg cgttgacatt gattattgcc tagttattaa tagtaatcaa     720

ttacggggtc attagttcat agcccatata tggagttccg cgttacataa cttacggtaa     780

atggcccgcc tggctgaccg cccaacgacc cccgcccatt gacgtcaata atgacgtatg     840

ttcccatagt aacgccaata gggactttcc attgacgtca atgggtggag tatttacggt     900

aaactgccca cttggcagta catcaagtgt atcatatgcc aagtacgccc cctattgacg     960

tcaatgacgg taaatggccc gcctggcatt atgcccagta catgacctta tgggactttc    1020

ctacttggca gtacatctac gtattagtca tcgctattac catggtgatg cggttttggc    1080

agtacatcaa tgggcgtgga tagcggtttg actcacgggg atttccaagt ctccacccca    1140

ttgacgtcaa tgggagtttg ttttggcacc aaaatcaacg ggactttcca aaatgtcgta    1200

acaactccgc cccattgacg caaatgggcg gtaggcgtgt acggtgggag gtctatataa    1260

gcagagctct ctggctaact agagaaccca ctgcttactg gcttatcgaa attactag      1318

atg gtg tct gag ctg atc aaa gaa aac atg cac atg aag ctg tac atg      1366
Met Val Ser Glu Leu Ile Lys Glu Asn Met His Met Lys Leu Tyr Met
1               5                   10                  15

gaa ggc acc gtg aac aac cac cac ttc aag tgc acc tcc gag ggc gag      1414
Glu Gly Thr Val Asn Asn His His Phe Lys Cys Thr Ser Glu Gly Glu

                    20                  25                  30

ggc aag cct tat gag ggc acc cag acc atg cgg atc aag gcc gtg gaa      1462
Gly Lys Pro Tyr Glu Gly Thr Gln Thr Met Arg Ile Lys Ala Val Glu
        35                  40                  45

ggc ggc cct ctg cct ttc gcc ttt gat atc ctg gcc acc tcc ttt atg      1510
Gly Gly Pro Leu Pro Phe Ala Phe Asp Ile Leu Ala Thr Ser Phe Met
    50                  55                  60

tac ggc tcc aag acc ttc atc aac cac acc cag ggc atc ccc gat ttc      1558
Tyr Gly Ser Lys Thr Phe Ile Asn His Thr Gln Gly Ile Pro Asp Phe
65                  70                  75                  80

ttc aag cag agc ttc ccc gag ggc ttc acc tgg gag aga gtg acc acc      1606
Phe Lys Gln Ser Phe Pro Glu Gly Phe Thr Trp Glu Arg Val Thr Thr
                85                  90                  95

tac gag gac ggc ggc gtg ctg acc gct acc cag gat acc tct ctc cag      1654
Tyr Glu Asp Gly Gly Val Leu Thr Ala Thr Gln Asp Thr Ser Leu Gln
            100                 105                 110

gac ggc tgc ctg atc tac aac gtg aag atc cgg ggc gtg aac ttc ccc      1702
Asp Gly Cys Leu Ile Tyr Asn Val Lys Ile Arg Gly Val Asn Phe Pro
        115                 120                 125

tcc aac ggc ccc gtg atg cag aag aaa acc ctg ggc tgg gag gcc agc      1750
Ser Asn Gly Pro Val Met Gln Lys Lys Thr Leu Gly Trp Glu Ala Ser
    130                 135                 140

acc gag aca ctg tat cct gct gac ggc ggc ctg gaa ggc aga gcc gat      1798
Thr Glu Thr Leu Tyr Pro Ala Asp Gly Gly Leu Glu Gly Arg Ala Asp
145                 150                 155                 160

atg gcc ctg aaa ctc gtg ggc gga ggc cac ctg atc tgt aac ctg aaa      1846
Met Ala Leu Lys Leu Val Gly Gly Gly His Leu Ile Cys Asn Leu Lys
                165                 170                 175

acc acc tac cgg tcc aag aag ccc gcc aag aac ctg aag atg ccc ggc      1894
Thr Thr Tyr Arg Ser Lys Lys Pro Ala Lys Asn Leu Lys Met Pro Gly
            180                 185                 190

gtg tac tac gtg gac cgg cgg ctg gaa cgg atc aaa gag gcc gac aaa      1942
Val Tyr Tyr Val Asp Arg Arg Leu Glu Arg Ile Lys Glu Ala Asp Lys
        195                 200                 205

gaa acc tac gtg gaa cag cac gag gtg gcc gtg gcc aga tac tgc gac      1990
Glu Thr Tyr Val Glu Gln His Glu Val Ala Val Ala Arg Tyr Cys Asp
    210                 215                 220

ctg cct agc aag ctg ggc cac aga tactagagac gggtggcatc cctgtgaccc      2044
Leu Pro Ser Lys Leu Gly His Arg
225                 230

ctccccagtg cctctcctgg ccctggaagt tgccactcca gtgcccacca gccttgtcct      2104

aataaaatta agttgcatca ttttgtctga ctaggtgtcc ttctataata ttatggggtg      2164

gaggggggtg gtatggagca aggggcaagt tgggaagaca acctgtaggg cctgcggggt      2224

ctattgggaa ccaagctgga gtgcagtggc acaatcttgg ctcactgcaa tctccgcctc      2284

ctgggttcaa gcgattctcc tgcctcagcc tcccgagttg ttgggattcc aggcatgcat      2344

gaccaggctc agctaatttt tgtttttttg gtagagacgg ggtttcacca tattggccag 2404

gctggtctcc aactcctaat ctcaggtgat ctacccacct tggcctccca aattgctggg 2464

attacaggcg tgaaccactg ctcccttccc tgtcctttac tagtaccggt ggtctctggc 2524

ggcggccgca ggaaccccta gtgatggagt tggccactcc ctctctgcgc gctcgctcgc 2584

tcactgaggc cgggcgacca aaggtcgccc gacgcccggg cggcctcagt gagcgagcga 2644

gcgcgcagct gcctgcaggg gcgcctgatg cggtattttc tccttacgca tctgtgcggt 2704

atttcacacc gcatacgtca aagcaaccat agtacgcgcc ctgtagcggc gcattaagcg 2764

cggcgggtgt ggtggttacg cgcagcgtga ccgctacact tgccagcgcc ctagcgcccg 2824

ctcctttcgc tttcttccct tcctttctcg ccacgttcgc cggctttccc cgtcaagctc 2884

taaatcgggg gctcccttta gggttccgat ttagtgcttt acggcacctc gaccccaaaa 2944

aacttgattt gggtgatggt tcacgtagtg ggccatcgcc ctgatagacg gtttttcgcc 3004

ctttgacgtt ggagtccacg ttctttaata gtggactctt gttccaaact ggaacaacac 3064

tcaaccctat ctcgggctat tcttttgatt tataagggat tttgccgatt tcggcctatt 3124

ggttaaaaaa tgagctgatt taacaaaaat ttaacgcgaa ttttaacaaa atattaacgt 3184

ttacaatttt atggtgcact ctcagtacaa tctgctctga tgccgcatag ttaagccagc 3244

cccgacaccc gccaacaccc gctgacgcgc cctgacgggc ttgtctgctc ccggcatccg 3304

cttacagaca agctgtgacc gtctccggga gctgcatgtg tcagaggttt tcaccgtcat 3364

caccgaaacg cgcgagacga aagggcctcg tgatacgcct atttttatag gttaatgtca 3424

tgataataat ggtttcttag acgtcaggtg gcacttttcg gggaaatgtg cgcggaaccc 3484

ctatttgttt atttttctaa atacattcaa atatgtatcc gctcatgaga caataaccct 3544

gataaatgct tcaataatat tgaaaaagga agagt atg agt att caa cat ttc 3597
Met Ser Ile Gln His Phe
        235

cgt gtc gcc ctt att ccc ttt ttt gcg gca ttt tgc ctt cct gtt ttt 3645
Arg Val Ala Leu Ile Pro Phe Phe Ala Ala Phe Cys Leu Pro Val Phe
    240                 245                 250

gct cac cca gaa acg ctg gtg aaa gta aaa gat gct gaa gat cag ttg 3693
Ala His Pro Glu Thr Leu Val Lys Val Lys Asp Ala Glu Asp Gln Leu
255                 260                 265                 270

ggt gca cga gtg ggt tac atc gaa ctg gat ctc aac agc ggt aag atc 3741
Gly Ala Arg Val Gly Tyr Ile Glu Leu Asp Leu Asn Ser Gly Lys Ile
                275                 280                 285

ctt gag agt ttt cgc ccc gaa gaa cgt ttt cca atg atg agc act ttt 3789
Leu Glu Ser Phe Arg Pro Glu Glu Arg Phe Pro Met Met Ser Thr Phe
            290                 295                 300

aaa gtt ctg cta tgt ggc gcg gta tta tcc cgt att gac gcc ggg caa 3837
Lys Val Leu Leu Cys Gly Ala Val Leu Ser Arg Ile Asp Ala Gly Gln

305                 310                 315

gag caa ctc ggt cgc cgc ata cac tat tct cag aat gac ttg gtt gag        3885
Glu Gln Leu Gly Arg Arg Ile His Tyr Ser Gln Asn Asp Leu Val Glu
    320                 325                 330

tac tca cca gtc aca gaa aag cat ctt acg gat ggc atg aca gta aga        3933
Tyr Ser Pro Val Thr Glu Lys His Leu Thr Asp Gly Met Thr Val Arg
335                 340                 345                 350

gaa tta tgc agt gct gcc ata acc atg agt gat aac act gcg gcc aac        3981
Glu Leu Cys Ser Ala Ala Ile Thr Met Ser Asp Asn Thr Ala Ala Asn
                355                 360                 365

tta ctt ctg aca acg atc gga gga ccg aag gag cta acc gct ttt ttg        4029
Leu Leu Leu Thr Thr Ile Gly Gly Pro Lys Glu Leu Thr Ala Phe Leu
            370                 375                 380

cac aac atg ggg gat cat gta act cgc ctt gat cgt tgg gaa ccg gag        4077
His Asn Met Gly Asp His Val Thr Arg Leu Asp Arg Trp Glu Pro Glu
        385                 390                 395

ctg aat gaa gcc ata cca aac gac gag cgt gac acc acg atg cct gta        4125
Leu Asn Glu Ala Ile Pro Asn Asp Glu Arg Asp Thr Thr Met Pro Val
    400                 405                 410

gca atg gca aca acg ttg cgc aaa cta tta act ggc gaa cta ctt act        4173
Ala Met Ala Thr Thr Leu Arg Lys Leu Leu Thr Gly Glu Leu Leu Thr
415                 420                 425                 430

cta gct tcc cgg caa caa tta ata gac tgg atg gag gcg gat aaa gtt        4221
Leu Ala Ser Arg Gln Gln Leu Ile Asp Trp Met Glu Ala Asp Lys Val
                435                 440                 445

gca gga cca ctt ctg cgc tcg gcc ctt ccg gct ggc tgg ttt att gct        4269
Ala Gly Pro Leu Leu Arg Ser Ala Leu Pro Ala Gly Trp Phe Ile Ala
            450                 455                 460

gat aaa tct gga gcc ggt gag cgt ggc tcg cgc ggt atc att gca gca        4317
Asp Lys Ser Gly Ala Gly Glu Arg Gly Ser Arg Gly Ile Ile Ala Ala
        465                 470                 475

ctg ggg cca gat ggt aag ccc tcc cgt atc gta gtt atc tac acg acg        4365
Leu Gly Pro Asp Gly Lys Pro Ser Arg Ile Val Val Ile Tyr Thr Thr
    480                 485                 490

ggg agt cag gca act atg gat gaa cga aat aga cag atc gct gag ata        4413
Gly Ser Gln Ala Thr Met Asp Glu Arg Asn Arg Gln Ile Ala Glu Ile
495                 500                 505                 510

ggt gcc tca ctg att aag cat tgg taa ctgtcagacc aagtttactc              4460
Gly Ala Ser Leu Ile Lys His Trp
                515

atatatactt tagattgatt taaaacttca tttttaattt aaaaggatct aggtgaagat      4520

cctttttgat aatctcatga ccaaaatccc ttaacgtgag ttttcgttcc actgagcgtc      4580

agaccccgta gaaaagatca aaggatcttc ttgagatcct ttttttctgc gcgtaatctg      4640

ctgcttgcaa acaaaaaaac caccgctacc agcggtggtt tgtttgccgg atcaagagct      4700

accaactctt tttccgaagg taactggctt cagcagagcg cagataccaa atactgtcct      4760

tctagtgtag ccgtagttag gccaccactt ca                                    4792

<210>  2
<211>  232
<212>  PRT
<213>  artificial

<220>
<223>  Synthetic Construct

<400>  2

Met Val Ser Glu Leu Ile Lys Glu Asn Met His Met Lys Leu Tyr Met
1               5                   10                  15

Glu Gly Thr Val Asn Asn His His Phe Lys Cys Thr Ser Glu Gly Glu
            20                  25                  30

Gly Lys Pro Tyr Glu Gly Thr Gln Thr Met Arg Ile Lys Ala Val Glu
        35                  40                  45

Gly Gly Pro Leu Pro Phe Ala Phe Asp Ile Leu Ala Thr Ser Phe Met
    50                  55                  60

Tyr Gly Ser Lys Thr Phe Ile Asn His Thr Gln Gly Ile Pro Asp Phe
65                  70                  75                  80

Phe Lys Gln Ser Phe Pro Glu Gly Phe Thr Trp Glu Arg Val Thr Thr
                85                  90                  95

Tyr Glu Asp Gly Gly Val Leu Thr Ala Thr Gln Asp Thr Ser Leu Gln
            100                 105                 110

Asp Gly Cys Leu Ile Tyr Asn Val Lys Ile Arg Gly Val Asn Phe Pro
        115                 120                 125

Ser Asn Gly Pro Val Met Gln Lys Lys Thr Leu Gly Trp Glu Ala Ser
    130                 135                 140

Thr Glu Thr Leu Tyr Pro Ala Asp Gly Gly Leu Glu Gly Arg Ala Asp
145                 150                 155                 160

Met Ala Leu Lys Leu Val Gly Gly Gly His Leu Ile Cys Asn Leu Lys
                165                 170                 175

Thr Thr Tyr Arg Ser Lys Lys Pro Ala Lys Asn Leu Lys Met Pro Gly
            180                 185                 190

Val Tyr Tyr Val Asp Arg Arg Leu Glu Arg Ile Lys Glu Ala Asp Lys

195                 200                 205

Glu Thr Tyr Val Glu Gln His Glu Val Ala Val Ala Arg Tyr Cys Asp
    210                 215                 220

Leu Pro Ser Lys Leu Gly His Arg
225                 230

<210>  3
<211>  286
<212>  PRT
<213>  artificial

<220>
<223>  Synthetic Construct

<400>  3

Met Ser Ile Gln His Phe Arg Val Ala Leu Ile Pro Phe Phe Ala Ala
1               5                   10                  15

Phe Cys Leu Pro Val Phe Ala His Pro Glu Thr Leu Val Lys Val Lys
            20                  25                  30

Asp Ala Glu Asp Gln Leu Gly Ala Arg Val Gly Tyr Ile Glu Leu Asp
        35                  40                  45

Leu Asn Ser Gly Lys Ile Leu Glu Ser Phe Arg Pro Glu Glu Arg Phe
    50                  55                  60

Pro Met Met Ser Thr Phe Lys Val Leu Leu Cys Gly Ala Val Leu Ser
65                  70                  75                  80

Arg Ile Asp Ala Gly Gln Glu Gln Leu Gly Arg Arg Ile His Tyr Ser
                85                  90                  95

Gln Asn Asp Leu Val Glu Tyr Ser Pro Val Thr Glu Lys His Leu Thr
            100                 105                 110

Asp Gly Met Thr Val Arg Glu Leu Cys Ser Ala Ala Ile Thr Met Ser
        115                 120                 125

Asp Asn Thr Ala Ala Asn Leu Leu Leu Thr Thr Ile Gly Gly Pro Lys
    130                 135                 140

Glu Leu Thr Ala Phe Leu His Asn Met Gly Asp His Val Thr Arg Leu
145                 150                 155                 160

Asp Arg Trp Glu Pro Glu Leu Asn Glu Ala Ile Pro Asn Asp Glu Arg
                165                 170                 175

Asp Thr Thr Met Pro Val Ala Met Ala Thr Thr Leu Arg Lys Leu Leu
            180                 185                 190

Thr Gly Glu Leu Leu Thr Leu Ala Ser Arg Gln Gln Leu Ile Asp Trp
        195                 200                 205

Met Glu Ala Asp Lys Val Ala Gly Pro Leu Leu Arg Ser Ala Leu Pro
    210                 215                 220

Ala Gly Trp Phe Ile Ala Asp Lys Ser Gly Ala Gly Glu Arg Gly Ser
225                 230                 235                 240

Arg Gly Ile Ile Ala Ala Leu Gly Pro Asp Gly Lys Pro Ser Arg Ile
                245                 250                 255

Val Val Ile Tyr Thr Thr Gly Ser Gln Ala Thr Met Asp Glu Arg Asn
            260                 265                 270

Arg Gln Ile Ala Glu Ile Gly Ala Ser Leu Ile Lys His Trp
        275                 280                 285

<210>  4
<211>  11
<212>  DNA
<213>  artificial

<220>
<223>  deletion ITR

<400>  4
tttgcccggg c                                                            11

<210>  5
<211>  18
<212>  DNA
<213>  artificial

<220>
<223>  primer

<400>  5
ggcggagttg ttacgaca                                                     18

<210>  6
<211>  19
<212>  DNA
<213>  artificial

<220>
<223>  primer

<400>  6

gggactttcc tacttggca                                                    19

<210>  7
<211>  6455
<212>  DNA
<213>  artificial

<220>
<223>  plasmid prepcap

<220>
<221>  gene
<222>  (51)..(1982)
<223>  Rep gene

<220>
<221>  gene
<222>  (1933)..(4140)
<223>  Cap gene

<220>
<221>  rep_origin
<222>  (4696)..(5315)
<223>  pBR322 origin

<220>
<221>  gene
<222>  (5597)..(6256)
<223>  CAT marker complement

<400>  7
atccttagct ttcgctaagg atgatttctg gaattcgcgg ccgcttctag atggcggggt      60

tttacgagat tgtgattaag gtccccagcg accttgacga gcatctgccc ggcatttctg     120

acagctttgt gaactgggtg gccgagaagg aatgggagtt gccgccagat tctgacatgg     180

atctgaatct gattgagcag gcacccctga ccgtggccga gaagctccag cgcgactttc     240

tgacggaatg gcgccgtgtg agtaaggccc cggaggccct tttctttgtg caatttgaga     300

agggagagag ctacttccac atgcacgtgc tcgtggaaac caccggggtg aaatccatgg     360

ttttgggacg tttcctgagt cagattcgcg aaaaactgat tcagagaatt taccgcggga     420

tcgagccgac tttgccaaac tggttcgcgg tcacaaagac cagaaatggc gccggaggcg     480

ggaacaaggt ggtggatgag tgctacatcc ccaattactt gctccccaaa acccagcctg     540

agctccagtg ggcgtggact aatatggaac agtatttaag cgcctgtttg aatctcacgg     600

agcgtaaacg gttggtggcg cagcatctga cgcacgtgtc gcagacgcag gagcagaaca     660

aagagaatca gaatcccaat tctgatgcgc cggtgatcag atcaaaaact tcagccaggt     720

acatggagct ggtcgggtgg ctcgtggaca aggggattac ctcggagaag cagtggattc     780

aggaggacca ggcctcatac atctccttca atgcggcctc caactcgcgg tcccaaatca     840

aggctgcctt ggacaatgcg ggaaagatta tgagcctgac taaaaccgcc cccgactacc     900

tggtgggcca gcagcccgtg gaggacattt ccagcaatcg gatttataaa attttggaac 960
taaacgggta cgatccccaa tatgcggctt ccgtctttct gggatgggcc acgaaaaagt 1020
tcggcaagag gaacaccatc tggctgtttg ggcctgcaac taccgggaag accaacatcg 1080
cggaggccat agcccacact gtgcccttct acgggtgcgt aaactggacc aatgagaact 1140
ttcccttcaa cgactgtgtg gacaagatgg tgatctggtg ggaggagggg aagatgaccg 1200
ccaaggtcgt ggagtcggcc aaagccattc tcggaggaag caaggtgcgc gtggaccaga 1260
aatgcaagtc ctcggcccag atagacccga ctcccgtgat cgtcacctcc aacaccaaca 1320
tgtgcgccgt gattgacggg aactcaacga ccttcgaaca ccagcagccg ttgcaagacc 1380
ggatgttcaa atttgaactc acccgccgtc tggatcatga ctttgggaag gtcaccaagc 1440
aggaagtcaa agactttttc cggtgggcaa aggatcacgt ggttgaggtg gagcatgagt 1500
tctacgtcaa aaagggtgga gccaagaaaa gacccgcccc cagtgacgca gatataagtg 1560
agcccaaacg ggtgcgcgag tcagttgcgc agccatcgac gtcagacgcg gaagcttcga 1620
tcaactacgc agacaggtac caaaacaaat gttctcgtca cgtgggcatg aatctgatgc 1680
tgtttccctg tagacaatgc gagagaatga atcagaactc aaatatctgc ttcactcacg 1740
gacagaaaga ctgtttagag tgctttcccg tgtcagaatc tcaacccgtt tctgtcgtca 1800
aaaaggcgta tcagaaactg tgctacattc atcatatcat gggaaaggtg ccagacgctt 1860
gcactgcctg cgatctggtc aatgtggatt tggatgactg catctttgaa caataaatga 1920
tttaaatcag gtatggctgc cgatggttat cttccagatt ggctcgagga cactctctct 1980
gaaggaataa gacagtggtg gaagctcaaa cctggcccac caccaccaaa gcccgcagag 2040
cggcataagg acgacagcag gggtcttgtg cttcctgggt acaagtacct cggacccttc 2100
aacggactcg acaagggaga gccggtcaac gaggcagacg ccgcggccct cgagcacgac 2160
aaagcctacg accggcagct cgacagcgga gacaacccgt acctcaagta caaccacgcc 2220
gacgcggagt ttcaggagcg ccttaaagaa gatacgtctt ttgggggcaa cctcggacga 2280
gcagtcttcc aggcgaaaaa gagggttctt gaacctctgg gcctggttga ggaacctgtt 2340
aagacggctc cgggaaaaaa gaggccggta gagcactctc ctgtggagcc agactcctcc 2400
tcgggaaccg gaaaggcggg ccagcagcct gcaagaaaaa gattgaattt tggtcagact 2460
ggagacgcag actcagtacc tgacccccag cctctcggac agccaccagc agccccctct 2520
ggtctgggaa ctaatacgat ggctacaggc agtggcgcac caatggcaga caataacgag 2580
ggcgccgacg gagtgggtaa ttcctcggga aattggcatt gcgattccac atggatgggc 2640
gacagagtca tcaccaccag cacccgaacc tgggccctgc ccacctacaa caaccacctc 2700
tacaaacaaa tttccagcca atcaggagcc tcgaacgaca atcactactt tggctacagc 2760
accccttggg ggtattttga cttcaacaga ttccactgcc acttttcacc acgtgactgg 2820

caaagactca tcaacaacaa ctggggattc cgacccaaga gactcaactt caagctcttt 2880

aacattcaag tcaaagaggt cacgcagaat gacggtacga cgacgattgc caataacctt 2940

accagcacgg ttcaggtgtt tactgactcg gagtaccagc tcccgtacgt cctcggctcg 3000

gcgcatcaag gatgcctccc gccgttccca gcagacgtct tcatggtgcc acagtatgga 3060

tacctcaccc tgaacaacgg gagtcaggca gtaggacgct cttcatttta ctgcctggag 3120

tactttcctt ctcagatgct gcgtaccgga aacaacttta ccttcagcta cacttttgag 3180

gacgttcctt tccacagcag ctacgctcac agccagagtc tggaccgtct catgaatcct 3240

ctcatcgacc aatatttgta ttacttgagc agaacaaaca ctccaagtgg taccaccacg 3300

cagagtcgac ttcagttttc tcaggccgga gcgagtgaca ttcgggacca gtctaggaac 3360

tggcttcctg gaccctgtta ccgccagcag cgagtatcaa agacatctgc ggataacaac 3420

aacagtgaat actcgtggac tggagctacc aagtaccacc tcaatggcag agactctctg 3480

gtgaatccgg gcccggccat ggcaagccac aaggacgatg aagaaaagtt ttttcctcag 3540

agcggggttc tcatctttgg gaagcaaggc tcagagaaaa caaatgtgga cattgaaaag 3600

gtcatgatta cagacgaaga ggaaatcagg acaaccaatc ccgtggctac ggagcagtat 3660

ggatccgtat ctaccaacct ccagagaggc aacagacaag cagctacagc tgatgtcaac 3720

acacaaggcg ttcttccagg catggtctgg caggacagag atgtgtacct tcaggggccc 3780

atctgggcaa agattccaca cacggacgga cattttcacc cctctcccct catgggtgga 3840

ttcggactta aacaccctcc tccacagatt ctcatcaaga acaccccggt acctgcgaat 3900

ccttcgacca ccttcagtgc ggcaaagttt gcttccttca tcacacagta ctccacggga 3960

caggtcagcg tggagatcga gtgggagctc cagaaggaaa acagcaaacg ctggaatccc 4020

gaaattcagt acacttccaa ctacaacaag tctgttaatg tggactttac tgtggacact 4080

aatggcgtgt attcagagcc tcgccccatt ggcaccagat acctgactcg taatctgtaa 4140

ttgcttgtta atcaataaac cgtttaattc gtttcagttg aactttggtc tctgcgtatt 4200

tctttcttat ctagtttcca tggctacgta gataagtagc atggcgggtt aatcattaac 4260

tactactaga gggaggggtg gagtcgtgac gtgaattacg tcatagggtt agggaggtcc 4320

tgtattagag gtcacgtgag tgttttgcga cattttgcga caccatgtgg tctcgctggg 4380

ggggggggcc cgagtgagca cgcagggtct ccattttgaa gcgggaggtt tgaactacta 4440

gtagcggccg ctgcagtccg gcaaaaaaac gggcaaggtg tcaccaccct gccctttttc 4500

tttaaaaccg aaaagattac ttcgcgttat gcaggcttcc tcgctcactg actcgctgcg 4560

ctcggtcgtt cggctgcggc gagcggtatc agctcactca aaggcggtaa tacggttatc 4620

cacagaatca ggggataacg caggaaagaa catgtgagca aaaggccagc aaaaggccag 4680

gaaccgtaaa aaggccgcgt tgctggcgtt tttccatagg ctccgccccc ctgacgagca 4740

tcacaaaaat cgacgctcaa gtcagaggtg gcgaaacccg acaggactat aaagatacca 4800

ggcgtttccc cctggaagct ccctcgtgcg ctctcctgtt ccgaccctgc cgcttaccgg 4860

atacctgtcc gcctttctcc cttcgggaag cgtggcgctt tctcatagct cacgctgtag 4920

gtatctcagt tcggtgtagg tcgttcgctc caagctgggc tgtgtgcacg aaccccccgt 4980

tcagcccgac cgctgcgcct tatccggtaa ctatcgtctt gagtccaacc cggtaagaca 5040

cgacttatcg ccactggcag cagccactgg taacaggatt agcagagcga ggtatgtagg 5100

cggtgctaca gagttcttga agtggtggcc taactacggc tacactagaa ggacagtatt 5160

tggtatctgc gctctgctga agccagttac cttcggaaaa agagttggta gctcttgatc 5220

cggcaaacaa accaccgctg gtagcggtgg tttttttgtt tgcaagcagc agattacgcg 5280

cagaaaaaaa ggatctcaag aagatccttt gatcttttct acggggtctg acgctcagtg 5340

gaacgaaaac tcacgttaag ggattttggt catgagatta tcaaaaagga tcttcaccta 5400

gatcctttta aattaaaaat gaagttttaa atcaatctaa agtatatatg agtaaacttg 5460

gtctgacagc tcgaggcttg gattctcacc aataaaaaac gcccggcggc aaccgagcgt 5520

tctgaacaaa tccagatgga gttctgaggt cattactgga tctatcaaca ggagtccaag 5580

cgagctcgat atcaaattac gccccgccct gccactcatc gcagtactgt tgtaattcat 5640

taagcattct gccgacatgg aagccatcac aaacggcatg atgaacctga atcgccagcg 5700

gcatcagcac cttgtcgcct tgcgtatagt atttgcccat ggtgaaaacg ggggcgaaga 5760

agttgtccat attggccacg tttaaatcaa aactggtgaa actcacccag ggattggctg 5820

agacgaaaaa catattctca ataaaccctt tagggaaata ggccaggttt tcaccgtaac 5880

acgccacatc ttgcgaatat atgtgtagaa actgccggaa atcgtcgtgg tattcactcc 5940

agagcgatga aaacgtttca gtttgctcat ggaaaacggt gtaacaaggg tgaacactat 6000

cccatatcac cagctcaccg tctttcattg ccatacgaaa ttccggatga gcattcatca 6060

ggcgggcaag aatgtgaata aaggccggat aaaacttgtg cttatttttc tttacggtct 6120

ttaaaaaggc cgtaatatcg agctgaacgg tctggttata ggtacattga gcaactgact 6180

gaaatgcctc aaaatgttct ttacgatgcc attgggatat atcaacggtg gtatatccag 6240

tgattttttt ctccatttta gcttccttag ctcctgaaaa tctcgataac tcaaaaaata 6300

cgcccggtag tgatcttatt tcattatggt gaaagttgga acctcttacg tgcccgatca 6360

actcgagtgc cacttgacgt ctaagaaacc attattatca tgacattaac ctataaaaat 6420

aggcgtatca cgaggcagaa tttcagataa aaaaa 6455